# EUROPEAN PATENT APPLICATION

(11) **EP 4 535 127 A1**
(43) Date of publication of application: **09.04.2025**
(21) Application number: 23815732.5
(22) Date of filing: 12.05.2023
(51) Int. Cl.: G06F 3/01, A61B 5/256, A61B 5/377, G06F 3/0346, G06F 3/038, G06F 3/04815

(54) **INFORMATION PROCESSING METHOD AND SYSTEM**

(30) Priority: 02.06.2022 JP 2022090375
(71) Applicant: Panasonic Intellectual Property Management Co., Ltd., Kadoma-shi, Osaka 571-0057 (JP)
(72) Inventor: NAGAMURA, Yoshiaki, Kadoma-shi, Osaka 571-0057 (JP)
(74) Representative: Grünecker Patent- und Rechtsanwälte PartG mbB
(86) International application number: PCT/JP2023/017984
(87) International publication number: WO 2023/233972

(57) **Abstract**

An information processing method for identifying a position that a worker visually recognizes with carefulness includes: acquiring as image data an image representing at least a part of a field of view of the worker at a predetermined time; acquiring as line-of-sight data a line-of-sight direction of the worker at the predetermined time; acquiring as electroencephalogram signal data an electroencephalogram signal of the worker in a time interval containing the predetermined time; detecting a predetermined waveform from the acquired electroencephalogram signal data; and determining a visually recognized position by the worker when the predetermined waveform occurs, based on the image data and the line-of-sight data.

## Description

### TECHNICAL FIELD

The present disclosure relates to an information processing method and system.

### BACKGROUND ART

Conventionally, a device is known that detects a user's field of view and line of sight to display information at a position that the user is viewing. For example, Patent Document 1 describes an interface that superimposes and displays information required by the user on an attention area that the user is paying attention to in his/her field of view.

### PATENT LITERATURE

Patent Document 1: JP 2014-93050 A

### SUMMARY OF INVENTION

### TECHNICAL PROBLEM

The interface described in Patent Document 1 detects only the line of sight of the user to identify the attention area. Therefore, the interface of Patent Document 1 cannot determine whether the user is carefully viewing the attention area.

An object of the present disclosure is to provide an information processing method and system for identifying a position that a worker visually recognizes with carefulness, based on an electroencephalogram signal measured from the worker.

### SOLUTION TO PROBLEM

An information processing method of the present disclosure for identifying a position that a worker visually recognizes with carefulness includes: acquiring as image data an image representing at least a part of a field of view of the worker at a predetermined time; acquiring as line-of-sight data a line-of-sight direction of the worker at the predetermined time; acquiring as electroencephalogram signal data an electroencephalogram signal of the worker in a time interval containing the predetermined time; detecting a predetermined waveform from the acquired electroencephalogram signal data; and determining a visually recognized position by the worker when the predetermined waveform occurs, based on the image data and the line-of-sight data.

### ADVANTAGEOUS EFFECT

According to the present disclosure, it is possible to provide an information processing method and system for identifying a position that a worker visually recognizes with carefulness, based on an electroencephalogram signal measured from the worker.

### BRIEF DESCRIPTION OF DRAWINGS

Fig. 1 is a block diagram of an example of a system for executing an information processing method according to an embodiment 1.
Fig. 2 is a flowchart showing a part of a generation stage of the information processing method according to the embodiment 1.
Fig. 3 is a flowchart showing another part of the generation stage of the information processing method according to the embodiment 1.
Fig. 4 is a graph showing an example of a reference waveform generated in the generation stage.
Fig. 5 is a flowchart showing a part of an attention location identification stage of the information processing method according to the embodiment 1.
Fig. 6 is a flowchart showing another part of the attention location identification stage of the information processing method according to the embodiment 1.
Fig. 7 is a flowchart showing a conveyance stage of the information processing method according to the embodiment 1.
Fig. 8 is a block diagram showing a modification of the system.
Fig. 9 is a block diagram of an example of a system for executing an information processing method according to an embodiment 2.
Fig. 10 is a flowchart showing a part of a generation stage of the information processing method according to the embodiment 2.
Fig. 11 is a flowchart showing another part of the generation stage of the information processing method according to the embodiment 2.
Fig. 12 is a block diagram of an environment detection device for executing the information processing method according to the embodiment 2.

### DETAILED DESCRIPTION

Embodiments according to the present disclosure will now be described with reference to the drawings. However, configurations described below are mere examples of the present disclosure. The present disclosure is not limited to the following embodiments, and even outside of these embodiments, various modifications can be made, depending on design, etc. without departing from technical ideas of the present disclosure.

### (Embodiment 1)

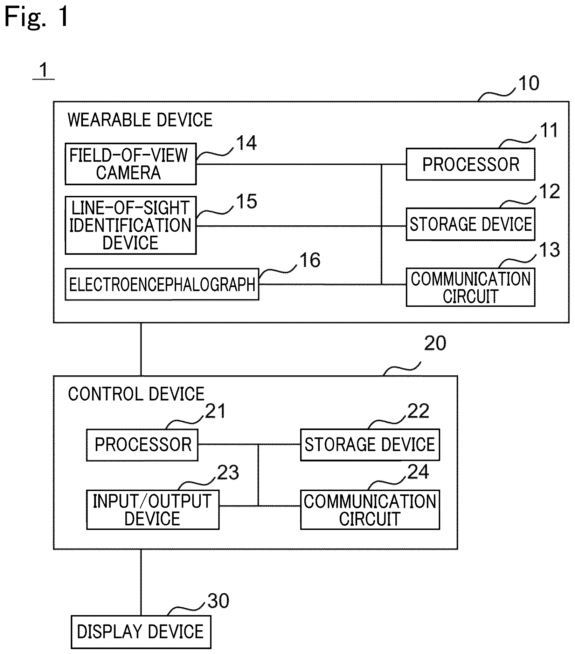

Fig. 1 is a block diagram of an example of a system 1 for executing an information processing method according to an embodiment 1 of the present disclosure. The information processing method according to this embodiment relates to a method for efficiently conveying a location to which a specific worker consciously or unconsciously pays attention during work to another worker. For example, the specific worker is a worker skilled in a specific work, while the another worker is a worker less skilled in the specific work than the specific worker (i.e., a worker less experienced than the specific worker). The location of attention is, for example, a position to which the specific worker's attention is drawn during work. While the specific worker is working at a work site, the information processing method according to this embodiment acquires image data representing at least a part of the specific worker's field of view, line-of-sight data representing the line-of-sight direction of the specific worker, and electroencephalogram signal data representing the electroencephalogram signal of the specific worker. Then, the information processing method determines in advance an electroencephalogram signal induced when the specific worker sees a predetermined event, and sets it as a reference waveform. The predetermined event indicates, for example, an event that has caught the specific worker's attention. In the method, the specific worker works again at the work site and image data, line-of-sight data, and electroencephalogram signal data are acquired. If the acquired electroencephalogram signal data contains a waveform similar to the reference waveform, the method determines the position where the specific worker was looking at when the waveform occurred based on the image data and the line-of-sight data, to determine the position as a location representing an event that attracted the specific worker's attention. By imparting a visual effect to the image data so as to emphasize the location, another worker different from the specific worker can efficiently understand the place to which the specific worker feels that he or she should pay attention by viewing the image data with visual effect imparted. In this manner, the information processing method can efficiently convey the location to which the specific worker consciously or unconsciously pays attention during work to another worker different from the specific worker.

As shown in Fig. 1, the system 1 includes a wearable device 10, a control device 20, and a display device 30. The wearable device 10 is attached to a specific worker. The wearable device 10 includes devices that can be worn on the head and eyes of the specific worker. The wearable device 10 includes a processor 11, a storage device 12, a communication circuit 13, a field-of-view camera 14, a line-of-sight identification device 15, and an electroencephalograph 16.

The processor 11 executes processing in the wearable device 10. The processor 11 includes a general-purpose processor such as a CPU or an MPU that executes a program to implement a predetermined function. The processor 11 is configured to be communicable with the storage device 12 and reads and executes an arithmetic program or the like stored in the storage device 12, to thereby implement various processes in the processor 11, such as a process of storing data acquired by the field-of-view camera 14 or the like. The processor 11 is not limited to a form where hardware resources and software cooperate to implement a predetermined function, and may be a hardware circuit designed dedicatedly to implement a predetermined function. That is, the processor 11 may be implemented by various processors such as a GPU, an FPGA, a DSP, an ASIC, etc. in addition to a CPU or MPU. Such a processor 11 may be configured, for example, by a signal processing circuit that is a semiconductor integrated circuit.

The storage device 12 is a storage medium capable of storing various pieces of information. The storage device 12 is implemented, for example, by a memory such as a DRAM, an SRAM, or a flash memory, an HDD, an SSD, or other storage device, or by an appropriate combination thereof. The storage device 12 stores programs for implementing various processes performed by the processor 11 as described above. The storage device 12 stores data acquired by the field-of-view camera 14, the line-of-sight identification device 15, and the electroencephalograph 16 as described below.

The communication circuit 13 is an interface device for connecting to other devices or systems via a communication line, either wired or wirelessly. The interface device is capable of performing communication in accordance with a wired communication standard, such as USB (registered trademark) or Ethernet (registered trademark). The interface device is capable of performing communication in accordance with a wireless communication standard, such as Wi-Fi (registered trademark), Bluetooth (registered trademark), or a mobile phone line.

The field-of-view camera 14 is an imaging device including an imaging element such as a CCD or a CMOS. In this embodiment, the field-of-view camera 14 is configured to capture an image in the direction where the specific worker is facing. The field-of-view camera 14 can acquire as image data an image (e.g., a moving image) that represents at least a part of the field of view of the specific worker. The acquired image data is stored in the storage device 12. The acquired image data may be stored in a storage device not shown that the field-of-view camera 14 has, and may be read out as necessary.

The line-of-sight identification device 15 is an imaging device including an imaging element such as a CCD or a CMOS. The line-of-sight identification device 15 is configured to capture an image of at least one eye of a specific worker. The line-of-sight identification device 15 is, for example, a device called an eye tracker or an eye tracking module and can identify or detect the line of sight of a specific worker wearing the device by infrared rays, to acquire line-of-sight data. The acquired line-of-sight data is stored in the storage device 12. The acquired line-of-sight data may be stored in a storage device not shown that the line-of-sight identification device 15 has, and may be read out as necessary.

The electroencephalograph 16 is a device capable of measuring electroencephalogram signals, and is, for example, an electroencephalograph. In this embodiment, the electroencephalograph 16 is configured to be attached to a specific worker to measure the electroencephalogram signals of the specific worker. The electroencephalograph 16 has a plurality of electrodes. The plurality of electrodes includes a measurement electrode and a reference electrode. The plurality of electrodes may include a ground electrode. The measurement electrode may be disposed at any of the positions defined by the International 10-20 System.

In the system 1 according to the present disclosure, the electroencephalograph 16 may be configured to measure an event-related potential as the electroencephalogram of the specific worker on which the electrodes are disposed. The measurement electrode may be disposed, for example, on the top of the specific worker's head. The reference electrode may be disposed, for example, on the left or right earlobe of the specific worker. The ground electrode may be disposed, for example, on the left or right earlobe of the specific worker. The potentials measured by the electroencephalograph 16 are not limited to event-related potentials, and can be other potentials.

The electroencephalograph 16 measures the potential difference between the plurality of electrodes as the electroencephalogram of the specific worker. The potential difference measured by the plurality of electrodes is stored as measured electroencephalogram data in the storage device 12. The electroencephalograph 16 may amplify the measured potential difference with an amplifier and store it as measured electroencephalogram data in the storage device 12. The measured potential difference may be stored in a storage device not shown that the electroencephalograph 16 has and may be read out as necessary.

The wearable device 10 transmits data acquired by the field-of-view camera 14, the line-of-sight identification device 15, and the electroencephalograph 16 to the control device 20 via the communication circuit 13 by wired or wireless communication.

The control device 20 is, for example, a computer. The control device 20 has a processor 21, a storage device 22, an input/output device 23, and a communication circuit 24. The control device 20 stores data received from the wearable device 10 into the storage device 22 via the communication circuit 24.

The processor 21 may be configured similarly to the processor 11 and executes processing in the control device 20. The processor 21 is configured to be communicable with the storage device 22 and reads and executes an arithmetic program or the like stored in the storage device 22, to thereby implement various types of processing in the processor 21, such as a process of generating a reference waveform and a process of identifying an attention location.

The storage device 22 may be configured similarly to the storage device 12 and stores programs for implementing the various processes performed by the processor 21 as described above.

The input/output device 23 functions as an input device for inputting information from a user and as an output device for outputting information to a user. The input/output device 23 includes one or more human-machine interface devices. The human-machine interface devices include, for example, input devices such as a keyboard, a pointing device (mouse, trackball, etc.), and a touch pad, output devices such as a display and a speaker, and input/output devices such as a touch panel.

The communication circuit 24 is configured similarly to the communication circuit 13.

The display device 30 is, for example, a display and is capable of displaying images, etc. output from the control device 20.

### (Information Processing Method)

Referring to Figs. 1 to 8, an information processing method according to the present disclosure will then be described. The information processing method according to the present disclosure can be divided into a plurality of stages. The plurality of stages includes a generation stage, an attention location identification stage, and a conveyance stage. The generation stage refers to a stage of generating a reference waveform indicative of a specific electroencephalogram signal induced when a specific worker sees a predetermined event. For example, the predetermined event refers to an event that has attracted the attention of the specific worker. Hereinafter, a position having an event that has attracted the attention of the specific worker is also referred to appropriately as an "attention location". The attention location identification stage refers to a stage of identifying an attention location by a reference waveform. The conveyance stage refers to a stage of conveying the attention location to another worker different from the specific worker. Note that the attention location may be a place that the specific worker consciously or unconsciously feels that attention should be paid to.

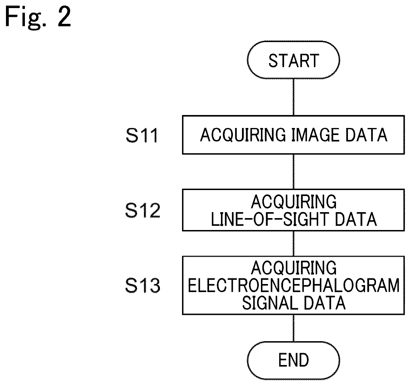

Fig. 2 is a flowchart showing a part of the generation stage of the information processing method according to the embodiment 1. The information processing method according to the present disclosure relates to a method for efficiently conveying to another worker a position having an event that attracts the attention of a specific worker. This method determines a position that the specific worker visually recognizes with carefulness, by an electroencephalogram signal measured from the specific worker. In the generation stage, this method determines a waveform (i.e., a reference waveform) of an electroencephalogram signal (hereinafter, referred to appropriately as a "specific electroencephalogram signal") induced when the specific worker visually recognizes an attention location. Fig. 2 is a flowchart showing a stage of collecting data for determining a reference waveform in the generation stage.

First, the specific worker moves to a work site and puts on the wearable device 10. Then, the specific worker switches on the wearable device 10. When the wearable device 10 is activated, the processor 11 of the wearable device 10 starts up the field-of-view camera 14, the line-of-sight identification device 15, and the electroencephalograph 16.

At step S11, the processor 11 acquires as image data an image representing at least a part of the field of view of the specific worker by the field-of-view camera 14. The processor 11 stores the acquired image data into the storage device 12. At step S12, by the line-of-sight identification device 15, the processor 11 acquires line-of-sight data of the specific worker to identify the line-of-sight direction. The processor 11 stores the acquired line-of-sight data into the storage device 12. At step S13, the processor 11 acquires electroencephalogram signal data representing the electroencephalogram signal of the specific worker by the electroencephalograph 16. The processor 11 stores the acquired electroencephalogram signal data into the storage device 12. When activating the wearable device 10, the specific worker starts work at the work site. The processor 11 of the wearable device 10 can acquire image data at a predetermined time, line-of-sight data at the predetermined time, and electroencephalogram signal data in a time interval containing the predetermined time, to store them in the storage device 12.

Then, when finishing the work at the work site, the specific worker switches off the wearable device 10.

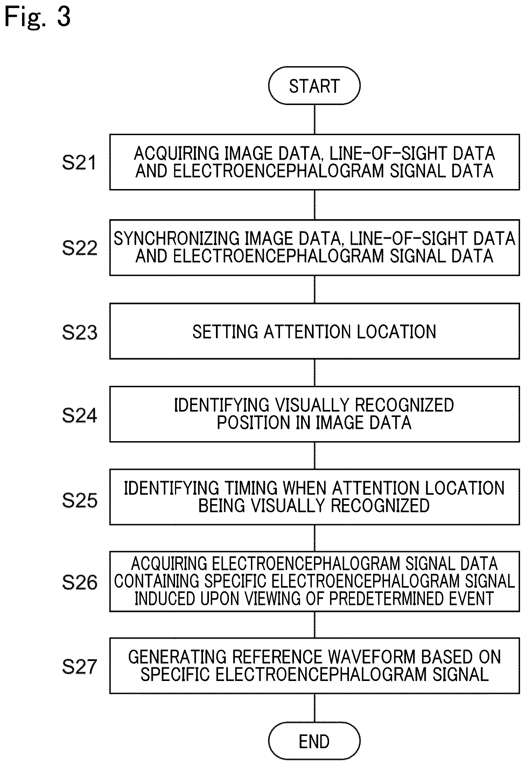

A stage after data collection in the generation stage will then be described. Fig. 3 is a flow chart showing another part of the generation stage of the information processing method according to the embodiment 1.

First, at step S21, the processor 21 of the control device 20 acquires image data, line-of-sight data, and electroencephalogram signal data via the communication circuit 24 from the storage device 12 of the wearable device 10. The processor 21 stores acquired data into the storage device 22.

Then, at step S22, the processor 21 synchronizes the acquired data with respect to time. The processor 21 synchronizes the image data, the line-of-sight data, and the electroencephalogram signal data with respect to time, thereby making it possible to associate the data with one another. The processor 21 can synchronize the data by using, for example, time information possessed by each piece of data. The processor 21 can thus acquire image data at a predetermined time, line-of-sight data at the predetermined time, and electroencephalogram signal data in a time interval containing the predetermined time, to store them in the storage device 22.

At step S23, the processor 21 sets a position that the specific worker determines to require attention, i.e., an attention location. The attention location may be set, for example, by the specific worker operating the input/output device 23 (e.g., a mouse) of the control device 20 while referring to image data stored in the storage device 22 of the control device 20. The attention location need not be set by the specific worker and may be set by another worker different from the specific worker.

At step S24, the processor 21 identifies a visually recognized position by the specific worker in the image data based on the image data and the line-of-sight data. This allows the processor 21 to identify the position (i.e., the visually recognized position) at which the specific worker was looking while working after step S13. For example, the processor 21 may combine the image data and the line-of-sight data to generate visually recognized position data in which the line-of-sight data is superimposed on the image data.

At step S25, the processor 21 identifies a timing when the specific worker visually recognizes the attention location. Then, at step S26, the processor 21 extracts an electroencephalogram signal at the timing, thereby acquiring electroencephalogram signal data containing a specific electroencephalogram signal representing the electroencephalogram signal of the specific worker when visually recognizing the attention location. That is, at step S26, the processor 21 acquires electroencephalogram signal data containing a specific electroencephalogram signal induced when the specific worker sees a predetermined event. Here, the predetermined event indicates an event that has attracted the attention of the specific worker. The attention location corresponds to a position having the predetermined event. In general, an electroencephalogram signal induced in the process of decision-making in an event-related potential is called P300, and a large potential change can occur approximately 300 ms to 500 ms after the occurrence of some trigger. In the method according to the present disclosure, the trigger corresponds to the timing when the specific worker sees the predetermined event (i.e., the timing when the specific worker visually recognizes the attention location). Accordingly, the processor 21 can acquire an event-related potential induced when a specific worker sees a predetermined event, for example, by using the electroencephalogram signal between 200 ms and 600 ms after that timing (i.e., 0 ms).

Naturally, the period to be acquired for the electroencephalogram signal is not limited to the above, and the processor 21 may use an electroencephalogram signal within another period, such as an electroencephalogram signal between 0 ms and 1000 ms, an electroencephalogram signal between 100 ms and 700 ms, or an electroencephalogram signal between 300 ms and 500 ms. At step S25, the processor 21 can identify a plurality of timings at which the specific worker visually recognized the attention location. Hence, at step S26, the processor 21 can acquire a plurality of specific electroencephalogram signals.

At step S27, the processor 21 calculates an additive average for the acquired specific electroencephalogram signals to generate a reference waveform. In general, the component of the electroencephalogram signal related to the event-related potential has a small potential change, so that desired waveform may not be acquired due to noise, e.g., of other components of the electroencephalogram signal. Hence, the processor 21 acquires a plurality of electroencephalogram signals related to the event-related potential to average the plurality of electroencephalogram signals, thereby reducing the influence of the components equivalent to noise to acquire a desired electroencephalogram signal. For example, the processor 21 can generate a more appropriate reference waveform by calculating an additive average for 10 or more specific electroencephalogram signals. The number of specific electroencephalogram signals to be averaged is not limited to 10 or more, and may be 13 or more, or 20 or more. It may also be 5 or more.

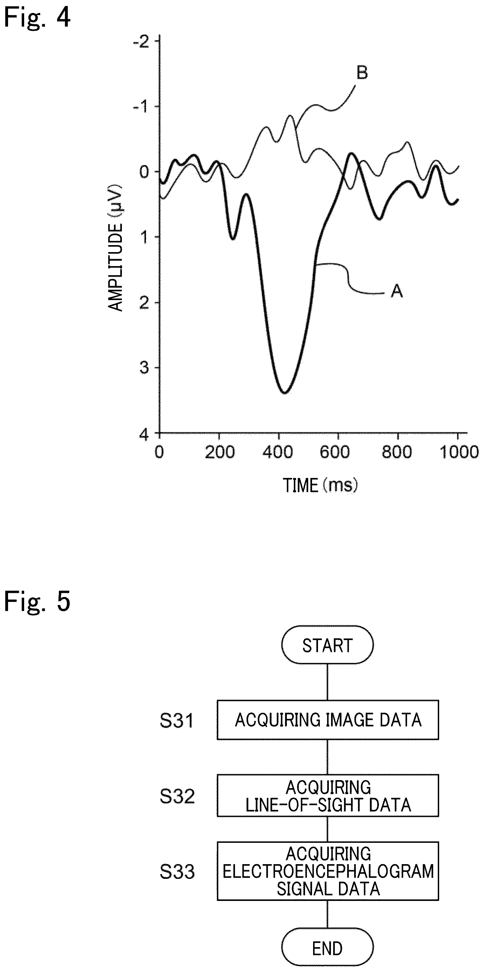

Fig. 4 is a graph showing an example of a reference waveform generated in the generation stage and a waveform of an electroencephalogram signal different from the reference waveform. In Fig. 4, waveform A shows the reference waveform, and waveform B shows the waveform of an electroencephalogram signal different from the reference waveform. The horizontal axis of Fig. 4 indicates time. The vertical axis of Fig. 4 indicates amplitude (i.e., potential), with the upper side indicating negative potential and the lower side indicating positive potential. As shown by waveform A of Fig. 4, the reference waveform undergoes a large potential change approximately 300 to 500 ms after the input of a trigger.

By carrying out the processing as described above, the processor 21 can generate a reference waveform that is defined based on a specific electroencephalogram signal that is induced when a specific worker sees a predetermined event.

In the above processing, the processor 21 sets the attention location after synchronizing the image data, the line-of-sight data, and the electroencephalogram signal data, but the timing to set the attention location is not limited thereto. For example, the attention location may be set after the processor 21 acquires the image data, the line-of-sight data, and the electroencephalogram signal data. The attention location may be set before the field-of-view camera acquires the image data at S11. In this case, for example, image data related to the work site where the specific worker performs the work is acquired in advance, and the specific worker or another worker operates the input/output device 23 while referring to the image data, so that the attention location can be set. When performing such a process, the attention location can be grasped in advance, so that the specific worker can be careful to visually recognize the attention location a plurality of times after step S13. Thus, the processor 21 can more reliably acquire the desired specific electroencephalogram signal a plurality of times from the electroencephalogram signal data.

Referring then to Fig. 5, the attention location identification step will be described. Fig. 5 is a flowchart showing a part of the attention location identification stage of the information processing method according to embodiment 1. The flowchart shown in Fig. 5 shows a stage of collecting image data, line-of-sight data, and electroencephalogram signal data in the attention location identification stage.

First, a specific worker moves to a work site and wears the wearable device 10. Then, the specific worker switches on the wearable device 10. When the wearable device 10 is activated, the processor 11 of the wearable device 10 starts up the field-of-view camera 14, the line-of-sight identification device 15, and the electroencephalograph 16.

At step S31, the processor 11 acquires as image data an image representing at least a part of the field of view of the specific worker by the field-of-view camera 14. The processor 11 stores the acquired image data into the storage device 12. At step S32, the processor 11 acquires line-of-sight data of the specific worker by the line-of-sight identification device 15 to identify the line-of-sight direction. The processor 11 stores the acquired line-of-sight data into the storage device 12. At step S33, the processor 11 acquires electroencephalogram signal data representing the electroencephalogram signal of the specific worker by the electroencephalograph 16. The processor 11 stores the acquired electroencephalogram signal data into the storage device 12. When activating the wearable device 10, the specific worker starts work at the work site. The processor 11 of the wearable device 10 can acquire image data at a predetermined time, line-of-sight data at the predetermined time, and electroencephalogram signal data in a time interval containing the predetermined time, to store them in the storage device 12.

Then, when finishing the work at the work site, the specific worker switches off the wearable device 10.

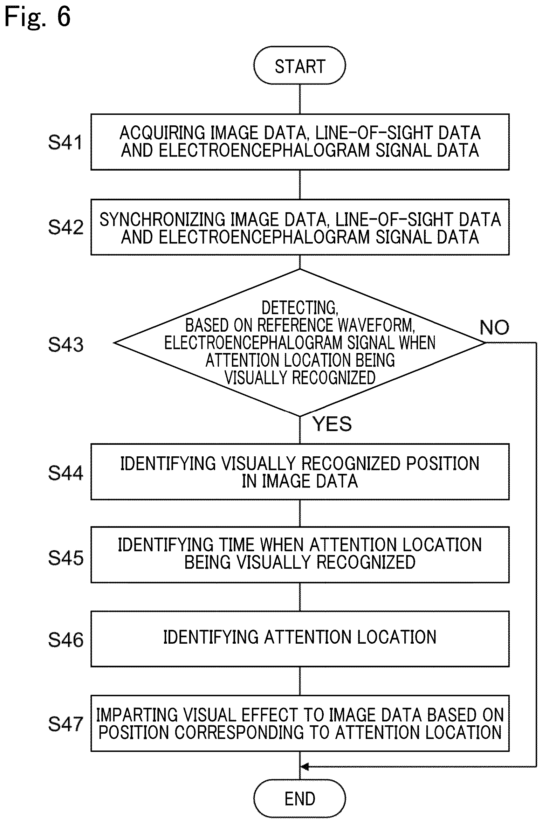

Stages after data collection in the attention location identification stage will then be described. Fig. 6 is a flowchart showing another part of the attention location identification stage of the information processing method according to the embodiment 1.

First, at step S41, the processor 21 of the control device 20 acquires image data, line-of-sight data, and electroencephalogram signal data via the communication circuit 24 from the storage device 12 of the wearable device 10. The processor 21 stores the acquired data into the storage device 22.

Next, at step S42, the processor 21 synchronizes the acquired data with respect to time. By synchronizing the image data, the line-of-sight data, and the electroencephalogram signal data with respect to time, the processor 21 can associate them with one another. The processor 21 can synchronize the data by using, for example, time information possessed by each piece of data. The processor 21 can thus acquire image data at a predetermined time, line-of-sight data at the predetermined time, and electroencephalogram signal data in a time interval containing the predetermined time, to store them in the storage device 22.

At step S43, the processor 21 detects, based on the reference waveform, an electroencephalogram signal of a predetermined waveform in the acquired electroencephalogram signal data induced when the specific worker sees a predetermined event (i.e., when the specific worker visually recognizes an attention location). More specifically, the processor 21 determines whether there is a waveform similar to the reference waveform in the acquired electroencephalogram signal data. The processor 21 detects a predetermined waveform from the electroencephalogram signal data when a part of the electroencephalogram signal contained in the acquired electroencephalogram signal data has a waveform corresponding to the reference waveform. The predetermined waveform is a waveform corresponding to the reference waveform in the electroencephalogram signal contained in the acquired electroencephalogram signal data. The processor 21 can determine whether there is a waveform similar to the reference waveform in the acquired electroencephalogram signal data by using, for example, a cross-correlation function (CCF) or a dynamic time warping (DTW). Since the CCF and the DTW are well known, a concrete description including a calculation method will be omitted. Note that the determination of whether there is a waveform similar to the reference waveform is not limited to the above method, and the processor 21 can determine using any method.

If an electroencephalogram signal induced when a specific worker sees a predetermined event is detected (S43: YES), that is, when it is determined that there is a waveform similar to the reference waveform, the processor 21 executes step S44, etc. If the electroencephalogram signal is not detected (S43: NO), the processor 21 terminates the process. The processor 21 may acquire a period during which the difference in potential between the reference waveform and a waveform showing a part of the electroencephalogram signal at a predetermined time is within a predetermined error, and may determine that the reference waveform and the waveform showing a part of the electroencephalogram signal are similar if the sum of the periods is equal to or longer than a predetermined time duration. In addition, the processor 21 may determine that the reference waveform and the waveform showing a part of the electroencephalogram signal are similar if the potential related to the waveform showing the part of the electroencephalogram signal is within a predetermined range. The predetermined range may be defined, for example, by the maximum and minimum values of the potential in the electroencephalogram signal (i.e., the specific electroencephalogram signal) used to generate the reference waveform. However, the method of determining the similarity by the processor 21 is not limited thereto.

For example, the processor 21 acquires measurement points at which the difference in potential between the reference waveform and a waveform showing a part of the electroencephalogram signal at a predetermined time is within a predetermined error. The processor 21 may then determine that the reference waveform and the waveform showing a part of the electroencephalogram signal are similar if the total number of measurement points is equal to or greater than a predetermined number. Alternatively, the processor 21 may calculate a DTW distance between the waveform of the reference waveform in a predetermined time interval and a waveform showing a part of the electroencephalogram signal and determine that the reference waveform and the waveform showing the part of the electroencephalogram signal are similar if the calculated value is equal to or less than a predetermined value. The predetermined time interval of the reference waveform is, for example, a period between 300 ms and 500 ms after the timing (0 ms) at which the specific worker sees the predetermined event, as described above.

At step S44, the processor 21 identifies a visually recognized position by the specific worker in the image data based on the image data and the line-of-sight data. This allows the processor 21 to identify a position (i.e., a visually recognized position) at which the specific worker was looking while working after step S33. For example, the processor 21 may combine the image data and the line-of-sight data to generate visually recognized position data in which the line-of-sight data is superimposed on the image data.

At step S45, the processor 21 identifies an occurrence time for the waveform similar to the reference waveform. If the processor 21 determines that the electroencephalogram signal data contains a waveform similar to the reference waveform in a plurality of time intervals, it identifies an occurrence time for each waveform.

At step S46, the processor 21 identifies a visually recognized position at the timing when a waveform similar to the reference waveform occurs. The processor 21 identifies, for example, a visually recognized position by the specific worker at the occurrence time identified at step S45, in the generated visually recognized position data. This allows the processor 21 to identify a position corresponding to the attention location in the image data based on the electroencephalogram signal data. If there are a plurality of occurrence times identified, the processor 21 identifies a visually recognized position by the specific worker for each time.

At step S47, the processor 21 imparts a visual effect to the image data based on the position identified at step S46. The processor 21 thereby generates effect-imparted image data. For example, the processor 21 imparts a visual effect to the image data so that the identified position can be distinguished therein. For example, the processor 21 may highlight the identified position in the image data by any image processing, such as by adding a color to the identified position, indicating it with an arrow, surrounding the identified position with a frame, or blinking the identified position, so that the identified position can be distinguished.

Thus, the processor 21 terminates the processing of the attention location identification step. In the above processing of the attention location identification stage, the attention location is identified by collecting image data, line-of-sight data, and electroencephalogram signal data and thereafter storing them in the control device 20 to perform the processes, but the processing is not limited thereto. For example, at the timing when performing the work (i.e., at and after S13), the specific worker may carry a mobile terminal having the same function as that of the control device 20 so that the processor of the mobile terminal may perform the processes described at steps S41 to S46. That is, the processor of the mobile terminal may identify the attention location while acquiring the image data, line-of-sight data, and electroencephalogram signal data. By performing the processing in this manner, the processor of the mobile terminal can identify the attention location in parallel with the work performed by the specific worker.

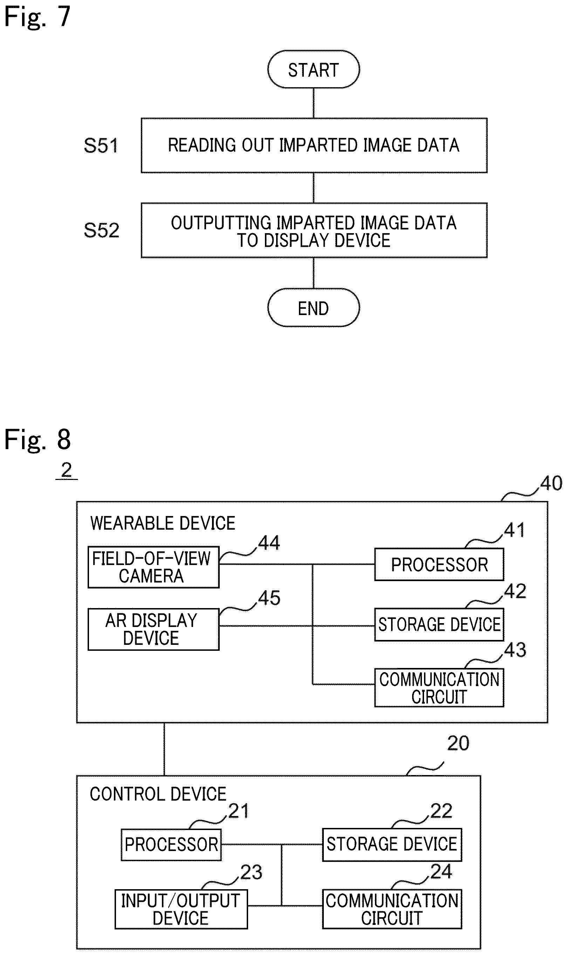

Referring then to Fig. 7, the conveyance stage will be described. Fig. 7 is a flowchart showing the conveyance stage of the information processing method according to the embodiment 1.

First, at step S51, the processor 21 reads out the effect-imparted image data stored in the storage device 22. At step S52, the processor 21 outputs the effect-imparted image data via the communication circuit 24 to the display device 30. The display device 30 displays the output effect-imparted image data. By viewing the displayed effect-imparted image data, another worker different from the specific worker can grasp which position in the image (i.e., the work site) the specific worker is paying attention to. This allows the another worker to learn the position that the specific worker consciously or unconsciously thinks he/she should pay attention to during the work.

The processor 21 thus terminates the processing of the conveyance stage. In the above processing of the conveyance stage, the processor 21 displays the effect-imparted image data on the display device 30, but the processing is not limited thereto.

For example, the conveyance process may be executed by another worker different from the specific worker wearing the wearable device 40 shown in Fig. 8.
Fig. 8 is a block diagram showing an example of a system 2 that is a modification of the system 1. The system 2 includes a wearable device 40 and the control device 20. Examples of the wearable device 40 include a head mounted display, a glasses-type device, etc. The wearable device 40 has a processor 41, a storage device 42, a communication circuit 43, a field-of-view camera 44, and an augmented reality (AR) display device 45.

The processor 41 may be configured similarly to the processor 21, and executes processing in the wearable device 40. The storage device 42 may be configured similarly to the storage device 22, and stores information on the above attention location in association with image data. The communication circuit 43 may be configured similarly to the communication circuit 24. The processor 41 receives, via the communication circuit 43, the attention location identified by the control device 20 and stores it in the storage device 42. The field-of-view camera 44 is an imaging device that may be configured similarly to the field-of-view camera 14. The AR display device 45 is a display device that can display an AR image so as to be superimposed on an image acquired by the field-of-view camera 44 based on the image. The AR display device 45 may be disposed in front of at least one eye of the another worker. For example, the processor 41 may identify a scene that the another worker is viewing based on an image acquired by the field-of-view camera 44, and display any AR image on the AR display device 45 so as to superimpose the above attention location on the scene.

By displaying in this manner, the processor 41 can display the AR image in an appropriate area depending on the scene seen by another worker who has moved to the work site, and can notify the another worker of the position that the specific worker feels should be careful about. The AR image can be generated so as to emphasize the position for the another worker, similar to the image processing at step S47.

The wearable device 40 may further include a line-of-sight identification device that identifies a line-of-sight direction of the another worker. By including the line-of-sight identification device, the processor 41 can more appropriately display an AR image on the AR display device 45 based on the line-of-sight direction of the another worker wearing the wearable device 40. For example, when the line of sight of the another worker is directed in a direction other than the attention location, the processor 41 may display an AR image in an area corresponding to the attention location on the AR display device 45. When the line of sight of the another worker is directed toward the attention location, the processor 41 may display an AR image in an area corresponding to the attention location on the AR display device 45.

### (Embodiment 2)

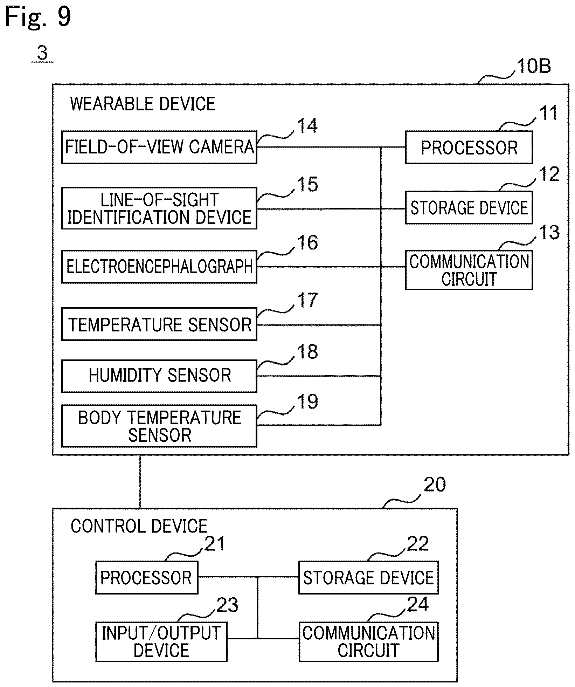

Referring to Figs. 9 to 12, an information processing method according to an embodiment 2 will be described. When a specific worker (e.g., an experienced worker) is working at a work site, the judgment ability of the specific worker may decline due to the external environment. The judgment ability may decline also due to the condition of the specific worker (e.g., the physical condition of the specific worker). If the specific worker works with the judgment ability declined, the work may be executed in a state where the judgment ability declines. Hence, the reliability of the work lowers. According to the information processing method according to the embodiment 2, however, the processor 21 of the control device 20 can determine whether the judgment ability of the specific worker declines due to the external environment, the condition of the specific worker, etc. Thus, according to the information processing method according to the embodiment 2, when it is determined that the judgment ability of the specific worker declines, an environment detection device 50 worn by the specific worker can notify the specific worker of the determination. Note that the information processing method according to the embodiment 2 is not limited to the specific worker and can similarly determine whether the judgment ability of another worker (e.g., an inexperienced worker) different from the specific worker declines.

Fig. 9 shows a block diagram of an example of a system 3 for executing the information processing method according to the embodiment 2. The system 3 includes a wearable device 10B and the control device 20. The wearable device 10B further includes a temperature sensor 17 and a humidity sensor 18 as external environment sensors, and a body temperature sensor 19 as a vital sensor, in addition to the wearable device 10 shown in Fig. 1. The external environment sensor is not limited to the temperature sensor, and may be any sensor such as the temperature sensor, the humidity sensor, a vibration sensor, a sound sensor, a barometric pressure sensor, or an odor sensor, or a combination of these sensors. The vital sensor is not limited to the body temperature sensor 19, and may be any sensor such as the body temperature sensor, a heart rate sensor, or a blood pressure sensor, or a combination of these sensors.

In the information processing method according to the embodiment 2, first, the generation stage of the information processing method according to the embodiment 1 is executed to generate a reference waveform for a specific worker. Preferably, the reference waveform is generated under work performed in an environment that places as little strain as possible on the specific worker. Here, the stressful environment may include low temperature, rain, vibration, strange odor, noise, etc.

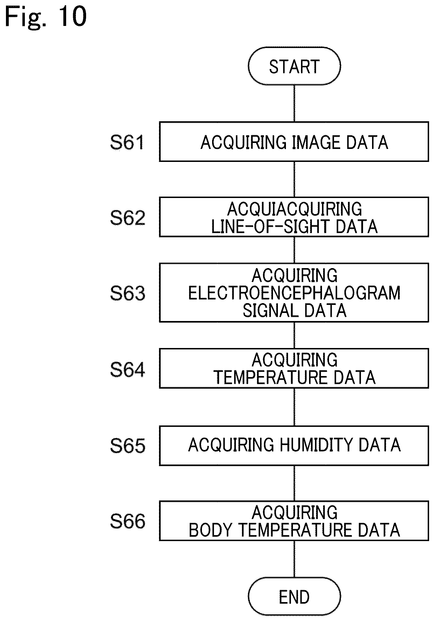

In the information processing method according to the embodiment 2, a specific worker then performs work at a work site while wearing the wearable device 10B. Fig. 10 is a flowchart showing a part of a generation stage of the information processing method according to the embodiment 2. First, the specific worker moves to the work site and wears the wearable device 10B. Then, the specific worker switches on the wearable device 10B. When the wearable device 10B is activated, the processor 11 of the wearable device 10B starts up the field-of-view camera 14, the line-of-sight identification device 15, the electroencephalograph 16, the temperature sensor 17, the humidity sensor 18, and the body temperature sensor 19.

At step S61, the processor 11 acquires as image data an image representing at least a part of the field of view of the specific worker using the field-of-view camera 14. The processor 11 stores the acquired image data into the storage device 12. At step S62, the processor 11 acquires line-of-sight data of the specific worker using the line-of-sight identification device 15, to identify a line-of-sight direction. The processor 11 stores the acquired line-of-sight data into the storage device 12. At step S63, the processor 11 acquires electroencephalogram signal data representing the electroencephalogram signal of the specific worker by the electroencephalograph 16. The processor 11 stores the acquired electroencephalogram signal data into the storage device 12.

At step 64, the processor 11 acquires a temperature at the work site as temperature data by the temperature sensor 17. The processor 11 stores the acquired temperature data into the storage device 12. At step 65, the processor 11 acquires a humidity at the work site as humidity data. The processor 11 stores the acquired humidity data into the storage device 12. At step 66, the processor 11 acquires the body temperature of the specific worker as body temperature data. The processor 11 stores the acquired body temperature data into the storage device 12. When activating the wearable device 10B, the specific worker starts working at the work site. The processor 11 of the wearable device 10B can acquire image data at a predetermined time, line-of-sight data at the predetermined time, and electroencephalogram signal data in a time interval containing the predetermined time, to store them in the storage device 12. The processor 11 can acquire temperature data at the predetermined time, humidity data at the predetermined time, and body temperature data at the predetermined time, to store them in the storage device 12.

Then, when finishing the work at the work site, the specific worker switches off the wearable device 10B.

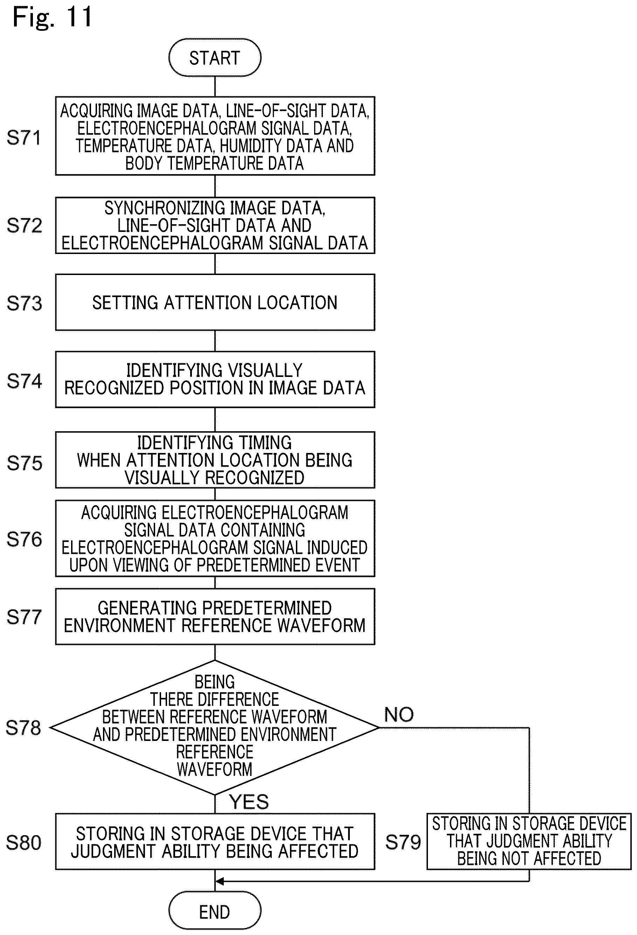

A description will then be given of a post-data collection stage of the generation stage of the method according to the embodiment 2. Fig. 11 is a flow chart showing another part of the generation stage of the information processing method according to the embodiment 2.

First, at step S71, the processor 21 of the control device 20 acquires image data, line-of-sight data, electroencephalogram signal data, temperature data, humidity data, and body temperature data via the communication circuit 24 from the storage device 12 of the wearable device 10B. The processor 21 stores the acquired data into the storage device 22.

At step S72, the processor 21 then synchronizes at least the image data, the line-of-sight data, and the electroencephalogram signal data among the acquired data with respect to time. The processor 21 may further synchronize other data with respect to time. The processor 21 can associate the image data, the line-of-sight data, and the electroencephalogram signal data with one another by synchronizing them with respect to time. The processor 21 can synchronize data by using, for example, time information possessed by each piece of data. The processor 11 can thus acquire image data at a predetermined time, line-of-sight data at the predetermined time, and electroencephalogram signal data in a time interval containing the predetermined time, to store them in the storage device 12. The processor 11 can acquire temperature data at the predetermined time, humidity data at the predetermined time, and body temperature data at the predetermined time, to store them in the storage device 12.

At step S73, the processor 21 sets an attention location. The attention location may be set, for example, by a specific worker operating the input/output device 23 (e.g., a mouse) of the control device 20 while referring to image data stored in the storage device 22 of the control device 20. The attention location need not be set by the specific worker, and may be set by another worker different from the specific worker.

At step S74, the processor 21 identifies a visually recognized position by the specific worker in the image data based on the image data and the line-of-sight data. This allows the processor 21 to identify a position (i.e., the visually recognized position) at which the specific worker was looking during work after step S66. For example, the processor 21 may combine the image data and the line-of-sight data to generate visually recognized position data in which the line-of-sight data is superimposed on the image data.

At step S75, the processor 21 identifies a timing at which the specific worker visually recognized the attention location. Then, at step S76, the processor 21 extracts an electroencephalogram signal at the timing, thereby acquiring electroencephalogram signal data representing the electroencephalogram signal of the specific worker when visually recognizing the attention location. That is, the processor 21 acquires electroencephalogram signal data containing an electroencephalogram signal induced when the specific worker views a predetermined event.

At step S77, the processor 21 calculates an additive average for a plurality of electroencephalogram signals acquired, to generate a predetermined environment reference waveform. The processor 21 associates the generated predetermined environment reference waveform with external environment information (i.e., temperature information and humidity information at the work site) and body temperature information of the specific worker, to store the association in the storage device 22. The temperature information, humidity information, and body temperature information may be average values over the work time, or may be maximum and minimum values. The temperature information, humidity information, and body temperature information may be values at the time when the above attention location is visually recognized.

At step S78, the processor 21 compares the previously prepared reference waveform with the predetermined environment reference waveform. If the processor 21 determines that there is no difference between the reference waveform and the predetermined environment reference waveform (S78: NO), at step S79, the processor 21 stores in the storage device that the judgment ability is not affected by the temperature information, humidity information, and body temperature information. If the processor 21 determines that there is a difference between the reference waveform and the predetermined environment reference waveform (S78: YES), at step S80, the processor 21 stores in the storage device that the judgment ability is affected by the temperature information, the humidity information, and the body temperature information.

For example, if the potential of the predetermined environment reference waveform is within a predetermined range, the processor 21 may determine that there is no difference between the reference waveform and the predetermined environment reference waveform. The predetermined range may be defined, for example, by the maximum and minimum values of the potential in the electroencephalogram signal (i.e., the specific electroencephalogram signal) used to generate the reference waveform. The method of determining whether there is a difference between the reference waveform and the predetermined environment reference waveform is not limited thereto. For example, the processor 21 may make a determination based on the difference in the average or median of the potential related to each waveform, or may make a determination based on a value calculated using the CCF or the DTW between the waveforms. The processor 21 may make a determination similar to step S43.

By performing the processing as described above, the processor 21 can determine whether the judgment ability of a specific worker is affected in a predetermined environment. By performing the above processing in various environments, the processor 21 can determine whether the judgment ability of a specific worker is affected in the various environments. For example, the processor 21 can store in the storage device, as judgment ability information, whether the judgment ability of a specific worker is affected in a predetermined environment.

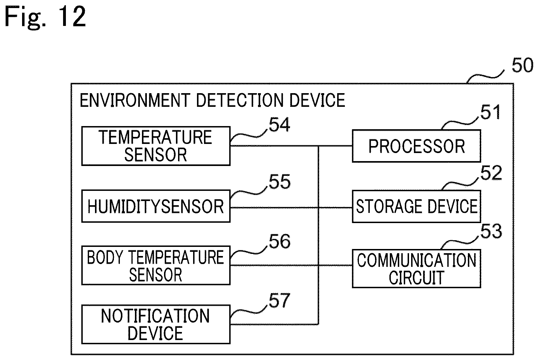

Fig. 12 shows a block diagram of the environment detection device 50 that can be worn by a specific worker to execute the information processing method according to the embodiment 2. The environment detection device 50 includes a processor 51, a storage device 52, a communication circuit 53, a temperature sensor 54, a humidity sensor 55, a body temperature sensor 56, and a notification device 57. The configuration of the environment detection device 50 is not limited to the above. For example, the environment detection device 50 can include any sensor that can be included in the wearable device 10B, such as a vibration sensor or a sound sensor.

The processor 51 may be configured similarly to the processor 21 and executes processing in the environment detection device 50. The storage device 52 may be configured similarly to the storage device 22 and stores judgment ability information. The temperature sensor 54 can detect ambient air temperature and conveys the air temperature as air temperature data to the processor 51. The humidity sensor 55 can detect ambient humidity and conveys the humidity as humidity data to the processor 51. The body temperature sensor 56 can detect a body temperature of a specific worker and conveys the body temperature as body temperature data to the processor 51. The notification device 57 can notify the specific worker of predetermined information based on an instruction from the processor 51. The notification device 57 is, for example, a speaker that can notify the specific worker of sound.

The processor 51 determines whether the judgment ability of the specific worker is affected in each piece of data acquired from the temperature sensor 54, the humidity sensor 55, and the body temperature sensor 56, based on the judgment ability information stored in the storage device 52. If it is determined that the judgment ability of the specific worker may decline, the processor 51 notifies the specific worker that the judgment ability of the specific worker may decline in the current environment, using the notification device 57. The specific worker wearing the environment detection device 50 can thus grasp that his or her own judgment ability may decline in the work site environment.

For example, if the judgment ability may decline when the humidity is high and the air temperature and the body temperature of the specific worker are low, the processor 51 may notify the specific worker that his/her judgment ability may decline when the data acquired from each of the sensors 54 to 56 is equal to or lower than a predetermined threshold. In this case, the predetermined threshold is the temperature for the temperature data and the body temperature data, and the humidity for the humidity data. When it is determined that the judgment ability of the specific worker will not decline based on the data acquired from each of the sensors 54 to 56, the processor 51 may notify the specific worker by using the notification device 57 that the judgment ability of the specific worker will not decline in the current environment.

The processor 51 may determine whether the judgment ability of the specific worker is affected based on only any of the temperature data, the humidity data, and the body temperature data. For example, if the judgment ability of the specific worker may decline when the temperature is low, the processor 51 may notify the specific worker by using the notification device 57 that the judgment ability of the specific worker may decline in the current environment based on only the temperature data.

As described above, the environment detection device 50 can include any sensors that can be included in the wearable device 10B. Hence, for example, if the wearable device 10B and the environment detection device 50 include a vibration sensor, the processor 51 can use the notification device 57 to notify the specific worker that the judgment ability of the specific worker may decline, based on whether a vibration has occurred.

### (Modification)

In the method according to the embodiments described above, the specific worker moves to a work site and acquires image data as a part of the field of view of the specific worker at the work site, but this is not limitative. For example, instead of the specific worker moving to the work site, an image corresponding to the work site may be displayed on a computer display in a building, and the processor 21 may use the image to identify a position that the specific worker feels should be careful about. The processor 21 may use the image to generate a reference waveform of an electroencephalogram signal that occurs when the specific worker visually recognizes an attention location.

In the above embodiment, the specific worker wears the wearable device 10 having the field-of-view camera 14, the line-of-sight identification device 15, and the electroencephalograph 16. However, the device worn by the specific worker is not limited thereto. For example, the specific worker may be equipped with the field-of-view camera 14, the line-of-sight identification device 15, and the electroencephalograph 16, instead of the wearable device 10. In this case, the control device 20 acquires image data, line-of-sight data, and electroencephalogram signal data from the field-of-view camera 14, the line-of-sight identification device 15, and the electroencephalograph 16, respectively.

In the above embodiment, the data acquired by the wearable device 10 is conveyed to the control device 20 so that the processor 21 of the control device 20 performs processes such as generating a reference waveform and identifying an attention location, but this is not limitative. For example, the wearable device 10 may convey each piece of data via the communication circuit 24 to a server device so that each process may be performed in the server device.

In the above embodiment, the processor 51 notifies the worker of the decline in the judgment ability based on environmental information such as the temperature or vital information such as body temperature when the worker is working, but the processing by the environment detection device 50 is not limited thereto. For example, the environment detection device 50 may further include an electroencephalograph, and the processor 51 may determine the decline in the judgment ability of the worker using an electroencephalogram signal in addition to the environmental information and vital information. Specifically, if the body temperature detected by the body temperature sensor 56 is a value that may decline the judgment ability of the specific worker and if the potential value of the acquired electroencephalogram signal is outside a predetermined range, the processor 51 may notify the specific worker that the judgment ability may decline by the notification device 57. As described above, the predetermined range may be defined by the maximum and minimum potential values in the electroencephalogram signal (i.e., the specific electroencephalogram signal) used to generate the reference waveform, but this is not limitative. Thereafter, if the body temperature detected by the body temperature sensor 56 changes to a value that is determined to cause no decline in judgment ability and if the potential value of the acquired electroencephalogram signal falls within a predetermined range, the processor 51 may notify the specific worker via the notification device 57 that the decline in judgment ability has been resolved.

### (SUMMARY OF ASPECTS)

As is apparent from the above description, the present disclosure includes the following aspects. In the following, reference numerals are given in parentheses only to clearly indicate the correspondence with the embodiments.

### (Aspect 1)

An information processing method for identifying a position that a worker visually recognizes with carefulness includes: acquiring as image data an image representing at least a part of a field of view of the worker at a predetermined time; acquiring as line-of-sight data a line-of-sight direction of the worker at the predetermined time; acquiring as electroencephalogram signal data an electroencephalogram signal of the worker in a time interval containing the predetermined time; detecting a predetermined waveform from the acquired electroencephalogram signal data; and determining a visually recognized position by the worker when the predetermined waveform occurs, based on the image data and the line-of-sight data. The information processing method can thereby identify the position where the worker carefully views based on the worker's electroencephalogram signal in the time interval containing the predetermined time measured from the worker.

### (Aspect 2)

In the information processing method of aspect 1, detecting the predetermined waveform may include detecting the predetermined waveform when a part of the electroencephalogram signal has a waveform corresponding to a reference waveform. Therefore, the information processing method can detect the predetermined waveform when the electroencephalogram signal contains a waveform corresponding to the reference waveform. Thus, the information processing method can detect the predetermined waveform when a waveform corresponding to the reference waveform occurs in the electroencephalogram signal, and determine the position visually recognized by the worker.

### (Aspect 3)

In the information processing method of aspect 2, the reference waveform may be defined based on a specific electroencephalogram signal measured in advance that is induced when the worker views a predetermined event. Consequently, the information processing method can detect the predetermined waveform based on the specific electroencephalogram signal induced when the worker views a predetermined event, and determine the position visually recognized by the worker.

### (Aspect 4)

In the information processing method of aspect 3, the predetermined event may be an event that attracts the worker's attention. As a result, the information processing method can detect a predetermined waveform based on a specific electroencephalogram signal induced when the worker visually recognizes a predetermined event and the worker's attention is attracted, and determine the position that the worker visually recognizes. Accordingly, the information processing method can determine the visually recognized position when the worker visually recognizes the event that has attracted the worker's attention.

### (Aspect 5)

The information processing method of any one of aspects 1 to 4 may further include conveying the visually recognized position to another worker different from the worker. Thereby, the information processing method can convey the position that the specific worker visually recognizes with carefulness to the another worker. Thus, the another worker can know the position that the specific worker visually recognizes with carefulness, and the information processing method can efficiently impart know-how from the specific worker to the another worker.

### (Aspect 6)

The information processing method of any one of aspects 1 to 5 may further include imparting a visual effect representing the visually recognized position onto the image. Therefore, the information processing method can generate an image representing the position carefully viewed by the worker. Thus, by the user referring to the image, the information processing method can easily and efficiently convey the visually recognized position to the user.

### (Aspect 7)

A system (1) includes: an imaging device (14) configured to acquire as image data an image representing at least a part of a field of view of a worker at a predetermined time; a line-of-sight identification device (15) configured to acquire as line-of-sight data a line-of-sight direction of the worker at the predetermined time; an electroencephalograph (16) configured to acquire as electroencephalogram signal data an electroencephalogram signal of the worker in a time interval containing the predetermined time; and a control device (20) having a storage device (22) and an processor (21), the storage device (22) configured to store the image data acquired from the imaging device, the line-of-sight data acquired from the line-of-sight identification device, and the electroencephalogram signal data acquired from the electroencephalograph, the processor (21) configured to execute instructions to detect a predetermined waveform from the electroencephalogram signal data and determining a visually recognized position of the worker when the predetermined waveform occurs, based on the image data and the line-of-sight data. Consequently, the system (1) can identify the position that the worker carefully views, based on the electroencephalogram signal of the worker in the time interval containing the predetermined time, measured from the worker.

### (Aspect 8)

The system (1) of aspect 7 may further include a display device (30) configured to display the image data and effect-imparted image data generated based on visually recognized position data representing the visually recognized position. This allows the system (1) to display the identified visually recognized position on the display device (30) and to communicate the visually recognized position to any user.

The system described in the present disclosure is implemented by cooperation between hardware resources, such as a processor and a memory, and software resources (computer programs).

### INDUSTRIAL APPLICABILITY

According to the present disclosure, there can be provided an information processing method and system for identifying a position that a worker visually recognizes with carefulness, based on an electroencephalogram signal measured from the worker, thus enabling suitable use in this type of industrial field.

### REFERENCE SIGNS LIST

- 1: system
- 10: wearable device
- 11: processor
- 12: storage device
- 13: communication circuit
- 14: field-of-view camera
- 15: line-of-sight identification device
- 16: electroencephalograph
- 17: temperature sensor
- 18: humidity sensor
- 19: body temperature sensor
- 20: control device
- 21: processor
- 22: storage device
- 23: input/output device
- 24: communication circuit
- 30: display device

## Claims

1. An information processing method for identifying a position that a worker visually recognizes with carefulness, comprising:
acquiring as image data an image representing at least a part of a field of view of the worker at a predetermined time;
acquiring as line-of-sight data a line-of-sight direction of the worker at the predetermined time;
acquiring as electroencephalogram signal data an electroencephalogram signal of the worker in a time interval containing the predetermined time;
detecting a predetermined waveform from the acquired electroencephalogram signal data; and
determining a visually recognized position by the worker when the predetermined waveform occurs, based on the image data and the line-of-sight data.

2. The information processing method according to claim 1, wherein detecting the predetermined waveform includes detecting the predetermined waveform when a part of the electroencephalogram signal has a waveform corresponding to a reference waveform.

3. The information processing method according to claim 2, wherein the reference waveform is defined based on a specific electroencephalogram signal measured in advance that is induced when the worker views a predetermined event.

4. The information processing method according to claim 3, wherein the predetermined event is an event that attracts the worker's attention.

5. The information processing method according to claim 1, further comprising:
conveying the visually recognized position to another worker different from the worker.

6. The information processing method according to claim 1, further comprising:
imparting a visual effect representing the visually recognized position onto the image.

7. A system comprising:
an imaging device configured to acquire as image data an image representing at least a part of a field of view of a worker at a predetermined time;
a line-of-sight identification device configured to acquire as line-of-sight data a line-of-sight direction of the worker at the predetermined time;
an electroencephalograph configured to acquire as electroencephalogram signal data an electroencephalogram signal of the worker in a time interval containing the predetermined time; and
a control device including
a storage device configured to store the image data acquired from the imaging device, the line-of-sight data acquired from the line-of-sight identification device, and the electroencephalogram signal data acquired from the electroencephalograph, and
a processor configured to execute instructions to detect a predetermined waveform from the electroencephalogram signal data and determining a visually recognized position by the worker when the predetermined waveform occurs, based on the image data and the line-of-sight data.

8. The system according to claim 7, further comprising:
a display device configured to display the image data and effect-imparted image data generated based on visually recognized position data representing the visually recognized position.
